# EUROPEAN PATENT SPECIFICATION

(11) **EP 0 396 612 B1**
(45) Date of publication and mention of the grant of the patent: **24.07.1996**
(21) Application number: 89901763.6
(22) Date of filing: 09.01.1989
(51) Int. Cl.: C12N 15/62, C12N 15/60, C07K 14/43, C07K 16/30

(54) **NOVEL PLASMID VECTOR WITH PECTATE LYASE SIGNAL SEQUENCE**
PLASMIDVEKTOR MIT PECTATLYASE-SIGNALSEQUENZ
NOUVEAU VECTEUR DE PLASMIDE AVEC SEQUENCE A FONCTION DE SIGNAL POUR PECTATE LYASE

(30) Priority: 11.01.1988 US 142039
(43) Date of publication of application: 14.11.1990
(73) Proprietor: Xoma Corporation, Berkeley California 94710 (US)
(72) Inventor: LEI, Shau-Ping, Los Angeles, CA 90064 (US); WILCOX, L., Gary, Malibu, CA 90265 (US)
(74) Representative: Sheard, Andrew Gregory
(86) International application number: US8900077
(87) International publication number: WO8906283

(56) References cited:
- EP-A- 0 133 321
- EP-A- 0 278 355
- WO-A-89/00999
- JOURNAL OF BACTERIOLOGY, vol. 169, no. 9, September 1987, Baltimore, MD (US); S.-P. LEI et al., pp. 4379-4383
- NUCLEIC ACIDS RESEARCH, vol. 12, July 1984; WATSON, pp. 5145-5146
- GENE, vol. 18, April 1982; EDENS et al., abstract
- T.M. ROBERTS, "Promoters, Structure, and Function", 1982, Praeger Publishers, New York, NY (US); pp. 452-461
- Gene, Volume 34, issued July 1985, (JOHNSTON et al) "High-Level Expression of M13 Gene II Protein from an Inducible Polycistronic Messenger RNA", see Abstract.
- Proceedings of the National Academy of Sciences, Volume 81, issued June 1984, (CABILLY et al) "Generation of Antibody Activity from Immunoglobulin Polypeptide Produced in Escherichia Coli", see Abstract.
- EMBO Journal 6, 3, 1987, pp. 823 - 831
- Gene 54, 1987, pp. 197 - 202
- The Journal of Biological Chemistry 267, 23, 1992, pp. 16712 - 16718
- Gene 134, 1993, pp. 223 - 227
- Journal of Bacteriology, vol. 168, November 1986; KEEN et al; abstract
- CHEMICAL ABSTRACTS, vol. 110, 13th February 1989, Columbus, Ohio (US); abstract no. 52329y, p. 187

## Description

This invention relates to externalization of foreign proteins from the cytoplasm of gram negative bacteria, in particular, E. coli, into the periplasmic space, and preferably into the extracellular fluid. The externalization can be made possible by the construction of a secretion vector, containing the signal peptide sequence for the pectate lyase gene derived from Erwinia carotovora, under the control of an E. coli-compatible promoter.

Typically, foreign proteins expressed in a unicellular host will not be secreted out of the cytoplasm, and therefore, isolation of the desired product requires disruption of the cell and separation from all the resulting cellular debris and unwanted native cytoplasmic proteins produced by the cell. Gram negative bacteria typically secrete their own proteins into a periplasmic space, an area outside the cytoplasm and in between the inner and outer membranes of the cell. Isolation of proteins from the periplasm is much simpler than the isolation from cytoplasm: the disruption of the cell wall is required, but rupture of the cell membrane is not necessary, and therefore avoids contamination of the foreign proteins with cytoplasmic debris. Isolation and separation of proteins secreted into the extracellular medium is of course a routine matter.

The advent of recombinant DNA technology has provided the ability to utilize single celled microorganisms as "factories" for the production of significant quantities of substantially pure proteins which may have value in therapeutic regimens or industrial processes. Briefly, recombinant DNA technology requires the insertion of specific DNA sequences into a DNA vehicle or vector, typically either a phage or a plasmid. In most cases, the DNA sequence inserted into the vector is one which is foreign to the vector, i.e., the inserted DNA sequence and the vector DNA sequence are derived from organisms which do not usually exchange genetic information in nature, or the inserted DNA may be partially or wholly synthetic. Methods of preparing these DNA vectors are now well known in the art.

For example, Cohen and Boyer, in U.S. Patent No. 4,237,224 describe the production of recombinant plasmids by the use of restriction enzymes and ligation; the resulting plasmids are then placed into a compatible unicellular host, by which process the foreign gene is introduced into the cell.

More specific techniques are set out in the following articles:

N T Keen *et al, Journal of Bacteriology,* **168 (2)**: 595-606 (November 1986) refers to the structure of two pectate lyase genes and their expression in *E. coli.*

WO-A-8 900 999, which was published on 09/02/89 and is thus comprised in the state of the art within the meaning of Art. 54 (3) EPC, discloses vectors that contain the pe1B sequence for use in the expression of three antibody fragments, namely L6 chimeric Fab, L6 chimeric light chain and L6 chimeric Fd.

EP-A-0 133 321 deals with DNA contructs in which a sequence coding for a signal sequence is immediately followed by a desired exogenous protein. The signal peptides of β-lactamase and alkaline phosphatase are given as specific examples.

M. Sjöström et al, *EMBO Journal* **6 (3)***:* 823 - 831 (1987) discusses swapping the signal peptides of various constructs. All of the signal peptides are E. coli derived.

C. Kato et al, *Gene* **54**: 197 - 202 (1987) discloses expression of a construct comprising a gene which encodes a protein and a signal peptide both of which are exogenous to E. coli. No pectate lyase sequences are mentioned.

M.E.E. Watson, *Nucleic Acids Research* **12 (13)**: 5145 - 5164 (1984) is a review where a large number of signal sequences are given. No pectate lyase sequences are mentioned.

L Edens *et al, Gene,* **18**: 1-12 (1982) refers to the cloning and expression of (prepro) thaumatin in *E. coli.*

S Cabilly *et al, Proc. Natl. Acad. Sci. USA,* **81**: 3273-3277 (June 1984) refers to the expression of heavy chains and/or light chains of an anti-carcinoembryonic antigen (CEA) antibody in *E. coli.*

A recombinant plasmid, in order to achieve the expression of foreign protein production, must be capable of autonomous replication in the host cell, and preferably will have a marker function which permits identification of the host cells which have been transformed. If all the proper replication, transcription and translation signals are correctly arranged in the plasmid, the foreign gene will be expressed in the transformed cells and the resulting progeny.

The progress of protein production by a cell does not end with its translation. Proteins produced within any given cell are not generally found throughout the cell, but rather tend to be compartmentalized; certain proteins are secreted, i.e., released from the cytoplasm, and others are not. The distribution of a given protein out of the cytoplasm is under the control of a signal sequence. Those cellular proteins which are destined to be secreted from the cell typically possess an N-terminal sequence of about 16 to 30 amino acid residues, most of which are hydrophobic. This hydrophobic region is essential for the signal function. The peptide which results from the translation of this region is believed to bind to the cell membrane; as translation of the remainder of the protein continues, the non-signal portion is drawn through the lipid bilayer. The signal sequence is then cleaved from the remainder of the protein by the enzyme signal peptidase.

The discerning of the function of signal sequences and peptides has prompted researchers to attempt to utilize the translation and transport mechanism of E. coli to produce and obtain proteins which are not normally produced by E. coli; these include both procaryotic and eucaryotic foreign proteins. The successful secretion of the proteins of interest, however, requires that the gene be associated with a signal sequence which is recognizable by a host cell promoter. Absent a compatible signal sequence, the foreign protein may be expressed by the host cell, but will not be distributed beyond the cytoplasm, into either the periplasmic space or the extracellular fluid. Clearly, a vector which could direct both the expression and secretion of a variety of foreign proteins in a host cell would provide a tremendous advantage in the use of recombinant technology to produce valuable proteins in large volume. The secretion vectors of the present invention provide such an advantage, surprisingly with a signal sequence which is not native to E. coli.

According to the present invention there is provided a recombinant vector suitable for transformation of a bacterial host, the vector containing a DNA sequence coding for a pectate lyase signal peptide adjacent a DNA sequence encoding a protein that is other than:
(a) pectate lyase or
(b) an antibody fragment which is L6 chimeric Fab, L6 chimeric light chain or L6 chimeric Fd;
wherein the vector can allow the protein to be expressed and to be transported to the periplasm of a gram-negative host or to be expressed and to be transported extracellularly relative to said host.

Further aspects of the present invention are disclosed in claims 2 to 11.

The vectors can be plasmid secretion vectors which, when used to transform bacterial host cells, enable the externalization of expressed foreign proteins. The plasmids can comprise a DNA sequence coding for a pectate lyase signal sequence of a gram-negative bacterium. These plasmids can in turn be used to prepare derivative plasmids containing a fragment consisting essentially of the signal sequence and a non-host protein gene sequence, and plasmids in which this fragment is positioned adjacent to a host-compatible promoter sequence. The latter plasmids, containing the promoter sequence, when used to transform a bacterial host cell, are capable of directing the translation and externalization of the foreign proteins by the host cell. The present invention thus provides a means by which foreign proteins can be produced in high volume by host cells without the necessity for cell lysis, and the attendant extensive purification procedures required to remove cytoplasmic contaminants. Isolation of expressed non-host proteins from the host would therefore be significantly facilitated if they can be externalized in this manner. The pectate lyase (pel) signal sequence functions well in the E. coli cell system, and the present secretion vectors have been shown to be capable of causing the secretion of virtually any protein used in the system. Secretion of the protein into the periplasmic space or extracellular environment is achieved by the insertion of a known gene sequence coding for a particular protein into the plasmid vector at some point after the pel signal sequence, and a suitable promoter sequence and then transforming a bacterial host cell with the secretion vector.

As disclosed herein, recombinant plasmids have been prepared which, when used to transform bacterial host cells, permit the secretion of foreign protein outside the cytoplasmic membrane of the host cell. The invention is based on the observation that when pectate lyase genes are cloned and expressed in E. coli, large quantities of pectate lyase are secreted either into the periplasmic space or culture fluid in which the bacterium is grown. This leads to the conclusion that the pectate lyase signal sequence is recognized and translated in a non-Erwinia bacterial system. When plasmids were prepared containing the signal sequence of the pel gene in combination with a foreign protein gene it was discovered that, in the presence of a host-compatible promoter, the signal sequence for the pel gene is capable of directing the distribution of the protein from the cytoplasm into the periplasmic space, or beyond the cell wall, into the extracellular environment. The secretion vectors of the present invention are particularly useful in that their utility is not limited to a single strain, but rather have been shown to function efficiently in a wide variety of readily available E. coli strains, all of which routinely secrete at least some of the recombinant protein directly into the culture medium. The plasmids have also been shown to be operable with a broad range of foreign gene sequences, both procaryotic and eucaryotic. Further, the proteins so produced have been confirmed to be secreted in their natural and proper configuration.

The present invention will now be described by way of example with reference to the accompanying drawings, wherein:
Figure 1 outlines the procedures used to create plasmids pSS1004, containing the pelB gene, and pSSl038, containing the pelB gene under control of the araBAD promoter. Details are provided in Example 2.1.1.
Figure 2A shows the sequence of the pelB signal peptide coding region, and the corresponding amino acid sequence. Figure 2B outlines the procedure used in construction of plasmids pING173, containing the pelB signal sequence, and plasmid pRR175 containing the pelB signal sequence downstream of the lac promoter. Details are provided in Example 2.2.
Figure 3 outlines the procedure used in construction of the plasmid pING177-1, containing the pelB signal sequence and the plant thaumatin gene under control of the araBAD promoter. Details are provided in Example 2.3.2.
Figure 4 outlines the procedures followed in construction of plasmid pING177-3, containing the plant thaumatin gene with the pre-thaumatin leader peptide sequence. Details are provided in Example 2.3.3.
Figure 5 outlines the procedures used in construction of the plasmids employed for the expression of the chimaeric mouse-human L6 antibody light chain. Details are provided in Example 2.4.2.
Figure 6 outlines the procedures used in construction of the plasmids employed for the expression of the chimaeric mouse-human L6 antibody heavy chain. Details are provided in Example 2.4.2.
Figure 7 outlines the procedures used in construction of the plasmids employed for the expression of the chimaeric mouse-human L6 antibody Fab. Details are provided in Example 2.4.2.
Figure 8 shows the restriction maps of the light chain and Fd gene cassette in pFK100, pFK101, pFK102, pFK103, pFK104. These plasmids were constructed as described in the text using plasmids outlined in Figure 6 and 7. The arrow indicates the direction of transcription from the lac promoter. E. carotovora and eukaryotic non-coding sequences are shown as open bars. The pelB leader sequence is crosshatched and the closed bar represents the antibody genes Fd and light chain (K).

### 1.1 IDENTIFICATION AND ISOLATION OF THE PECTATE LYASE GENE

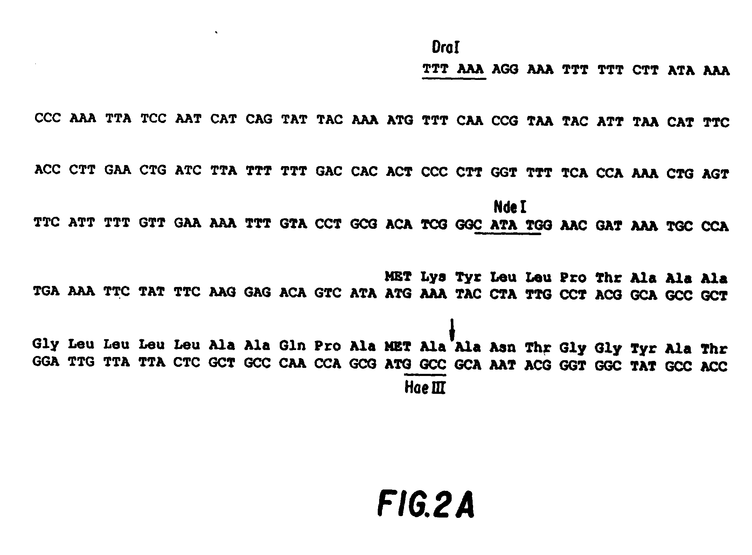

Pectate lyase is one of the enzymes which catalyzes the hydrolysis of polygalacturonic acid, and is found in a number of phytopathogenic bacteria and fungi. Three pectate lyases, referred to as PLa, PLb and PLc, have been identified from the bacterium Erwinia carotovora, and similar enzymes are also known in the bacteria Erwinia chrysanthemi (Keen et al., J. Bacteriol. 168: 595-606, 1986) and Pseudomonas fluorescens, as well as the fungus Rhizoctonia solani. The genes from both E. carotovora (Lei et al., Gene 35: 63-70, 1985) and E. chrysanthemi (Keen et al., supra) have been isolated. In the present case, the pelB gene from E. carotovora was used as a source of the pectate lyase signal sequence. Plasmid pSS1004 (Lei et al., J. Bacteriol. 169: 4379-4383, 1987) contains the E. carotovora pelB gene. The restriction sites around the signal sequence were identified based on the DNA sequence of the gene. The N-terminal amino acid sequence was also determined to confirm the location of the leader peptide. Treatment of the pSS1004 plasmid with HaeIII and EcoRI restriction enzymes produced a fragment containing the leader sequence. The gene sequence and the corresponding amino acid sequence of the pelB signal peptide is shown in Figure 2A. Alternatively, the peptide sequence may be chemically synthesized by known methods of peptide synthesis.

### 1.2. CONSTRUCTION OF SECRETION VECTORS

Once the fragment containing the appropriate signal sequence has been identified and isolated, it is then inserted into a cloning vehicle, preferably a plasmid. The present vectors are prepared in accordance with the general principles of vector construction known in the art. As used in the present specification and claims, "secretion" refers to transport of the protein into the periplasmic space, and "excretion" refers to the transport of the protein into the culture medium.

In order to eventually achieve transcription and translation of the inserted gene, the gene must be placed under the control of a promoter compatible with the chosen host cell. A promoter is a region of DNA at which RNA polymerase attaches and initiates transcription. The promoter selected may be any one which has been isolated from or is capable of functioning in the host cell organism. For example, E. coli has numerous promoters such as the lac or recA promoter associated with it, its bacteriophages or its plasmids. Also, phage promoters, such as the λ phage P_{L} and P_{R} promoters may be used to direct high level production of the products coded for having the segments of DNA adjacent to it. The products may be natural, synthetic or recombinant.

An initiation signal is also necessary in order to attain efficient translation of the gene. For example, in E. coli mRNA, a ribosome binding site includes the translational start codon (AUG or GUG) and another sequence complementary to the bases of the 3'-end of 16S ribosomal RNA. Several of these latter sequences (Shine-Dalgarno or S-D) have been identified in E. coli and other suitable host cell types. Any SD-ATG sequence which is compatible with the host cell system can be employed. These include, but are not limited to, the cro gene or N gene of coliphage lambda, or the E. coli tryptophan E, D, C, B or A genes.

A number of methods exist for the insertion of DNA fragments into cloning vectors in vitro. DNA ligase is an enzyme which seals single-stranded nicks between adjacent nucleotides in a duplex DNA chain; this enzyme may therefore be used to covalently join the annealed cohesive ends produced by certain restriction enzymes. Alternately, DNA ligase can be used to catalyze the formation of phosphodiester bonds between blunt-ended fragments. Finally, the enzyme terminal deoxynucleotidyl transferase may be employed to form homopolymeric 3'-single-stranded tails at the ends of fragments; by addition of oligo (dA) sequences to the 3'-end of one population, and oligo (dT) blocks to 3'-ends of a second population, the two types of molecules can anneal to form dimeric circles. Any of these methods may be used to ligate the gene segment, promoter and other control elements into specific sites in the vector. Thus, the coding sequence for a particular protein is ligated into the chosen vector in a specific relationship to the vector promoter and control elements and to the pel signal sequence, so that the protein gene sequence is in the correct reading frame with respect to the vector ATG sequence. The vector employed will typically have a marker function, such as ampicillin resistance or tetracycline resistance, so that transformed cells can be identified. The vector may be any of the known expression vectors or their derivatives; among the most frequently used are plasmid vectors such as pBR322, pAC105, pVA5, pACYC177, PKH47, pACYC184, pUB110, pMB9, pBR325, ColEl, pSC101, pBR313, pML21, RSF2124, pCR1 or RP4; bacteriophage vectors such as lambda gt11, lambda gt-WES-lambdaB, Charon 28, Charon 4A, lambda gt-1-lambda BC, lambda-gt-l-lambda B, M13mp7, M13mp8, M13mp9; SV40 and adenovirus vectors, and yeast vectors.

The present invention preferably employs a plasmid as the cloning vector. For example, the approach taken in the present examples, in the cloning of a number of different proteins in E. coli, was to first separately clone the PLb signal sequence. This was achieved by isolation of the HaeIII + ECORI digest fragment from plasmid pSS1004 which contains the entire pectate lyase B sequence. This fragment is then ligated into a pBR322 plasmid, which has been digested with SspI, ligated with an SstI linker, and then digested with EcoRI. Thus formed is the plasmid pINGl73 containing the pelB signal sequence. The pING173 plasmid is then used as a cloning vehicle for the pelB signal sequence, which may be subsequently ligated into a plasmid containing the sequence of the protein of interest. The plasmid so prepared contains a hybrid gene consisting of the pelB signal sequence adjacent to the relevant protein gene sequence; an appropriate promoter sequence may then be inserted at the 5'- terminus of the hybrid gene thus creating the final expression vector. Alternately, a plasmid containing the promoter sequence and the pelB signal sequence can first be prepared, and then the protein gene sequence may be inserted downstream of the promoter-signal sequences. Promoters which have proven particularly useful in the present invention are the Salmonella typhimurium araBAD and E. coli lac promoters in combination with an E. coli host system. However, any other suitable promoter which is compatible with the host system of choice may also be employed. Prepared by the foregoing method are plasmid expression vectors containing the genes for thaumatin, and L6 chimeric Fab, but it will be readily apparent to one skilled in the art that genes for any type of protein may be used in the present vectors and methods.

### 1.3. TRANSFORMATION

Once a vector with the appropriate promoter has been obtained, the plasmid may be used to transform a host bacterium. For example, virtually every strain of E. coli treated has been transformable (albeit with variable rates of transformation), so that the present invention is not limited to use with a single strain. The majority of techniques employed in transformation depend upon the observations of Mandel and Higa (J. Mol. Biol 53: 159-162, 1970) that uptake of vector DNA is substantially enhanced by treatment of bacterial cells with calcium chloride. Vector DNA is exposed to the calcium-chloride treated bacteria for a short period, usually no more than 12-24 hours. The exposed cells are then screened for transformants; this is achieved by selecting for those bacteria expressing the appropriate marker characteristics from the vector. For example, if a plasmid having a marker for tetracycline resistance has been employed, the bacteria are grown in a medium containing tetracycline, and those bacteria which continue to grow are recovered as putative transformants. These bacteria may then be further screened for the production of the protein of interest.

### 1.4. ISOLATION OF THE GENE PRODUCT

The present invention permits the gene product to be isolated from either the periplasmic space or the surrounding growth medium. The location of the expressed protein appears to be dependent on the particular strain utilized as the host. One of the most unexpected aspects of the present invention is that all strains tested were capable of excreting at least some of the recombinant product into the culture medium. The principal distinction between strains is the ratio of the amount of excreted product (i.e., that which is transported into the medium) to the amount secreted into the periplasmic space. Among those E. coli strains which show high levels of excretion are MC1061, JM103 and 706. While proteins which are excreted into the culture fluid are readily isolatable therefrom by known protein recovery techniques, the recovery of protein localized in the periplasmic space requires penetration of the cell wall in order to achieve release of the proteins without disruption of the cytoplasmic membrane. One technique of removal of periplasmic proteins is that originally described by Zinder and Arndt (PNAS USA 42: 586-590, 1956), which involves removal of the cell wall. Briefly, the cells are treated with egg albumin, which contains lysozyme, producing cellular spheres, or spheroplasts, which have large portions of the cell wall removed. Periplasmic proteins may also be isolated by a mechanism which does not require removal of the cell wall, but instead causes release of the proteins. Cells are placed in a hypertonic sucrose medium containing ethylene diamine tetraacetic acid (EDTA); this medium causes the cells to lose water and shrink, so that the cytoplasmic membrane draws away from the cell wall. The cells are then placed in a magnesium chloride solution which induces an osmotic shock: the osmotic pressure outside the cell decreases, causing water to rush into the cell, which swells the cell and causes the expulsion of periplasmic proteins beyond the outer membrane. Variations in the foregoing procedures will be readily apparent to one skilled in the art.

The present secretion vectors have been used successfully to secrete a variety of different types of proteins in E. coli. The following examples illustrate detailed methods of preparation of a number of different types of plasmids in accordance with the disclosure provided above. Where examples are not within the scope of the present claims, they are not examples of the present invention, but are provided for information purposes.

### 2.1. EXPRESSION OF PECTATE LYASE B

### 2.1.1. PLASMID CONSTRUCTION

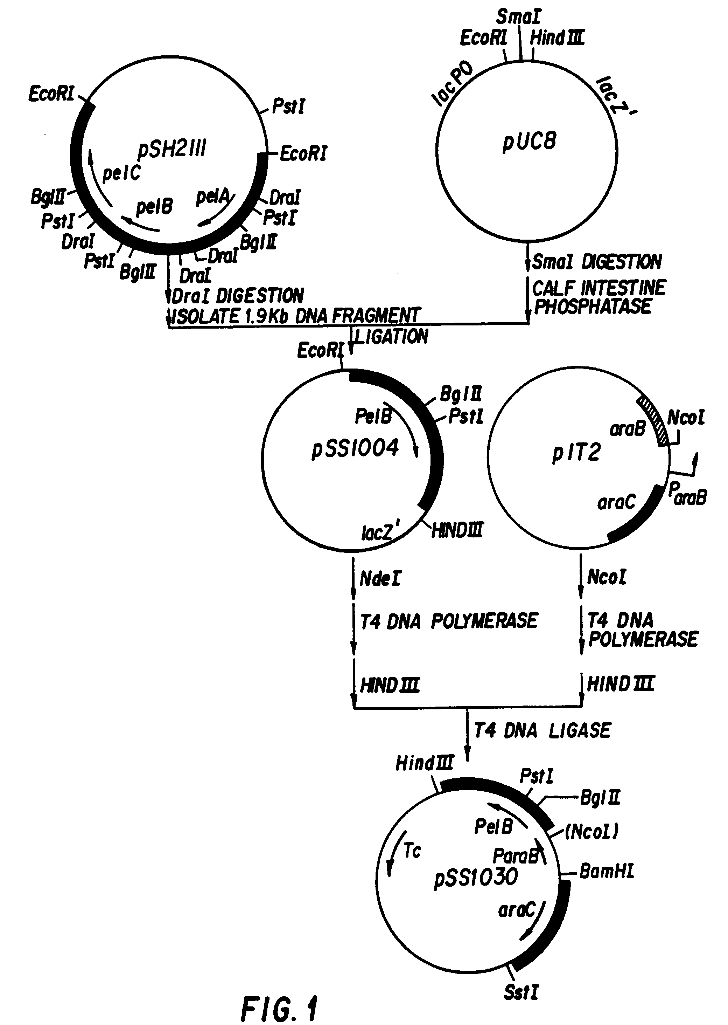

Plasmid psH2111 (Lei et al., Gene 35: 63-70, 1985) contains the pectate lyase genes from Erwinia carotovora. The pelB gene is positioned between two DraI restrictions sites; isolation of the gene is achieved by digestion of plasmid pSH2111 with DraI and identification of a 1.9 kilobase fragment on an agarose gel. The isolated fragment was then ligated into plasmid pUC8 which had been digested with SmaI. The resultant plasmid is pSS1004 (see Fig. 1).

The plasmid pSS1004 was then treated with NdeI T₄ DNA polymerase and Hind III, and the resultant fragment ligated into plasmid pIT2, which had been digested with NcoI, T₄ DNA polymerase and HindIII, to produce plasmid pSS1038 (Figure 1). The plasmid pIT2 contains both the Salmonella typhimurium araBAD promoter and the araC gene (Johnson et al. - Gene 34: 137-145, 1985). The plasmid was then used to transform E. coli strain 706. The expression of the pelB gene on plasmid pSSl038 was thus expected to be under the control of the araBAD promoter and turned on by the presence of arabinose in the growth medium.

### 2.1.2. EXCRETION AND PURIFICATION OF PLB

E. coli cell 706 (F⁻, pro, thr, leu, argH, his, lac, phoSt, rpsL, lky-207) carrying plasmid pSSl038 was used to characterize the production and excretion of PLb. The E. coli cells were grown in TYE (1.5% tryptone, 1% yeast extract and 0.5% NaCl), incubated at 37°C, at log phase of growth (around O.D.₅₄₀=0.6), 1% of arabinose was added to the growth medium to turn on the araBAD promoter and start producing PLb. After four hours of induction(O.D.₅₄₀ was about 2.5), the culture broth was centrifuged and PLb was directly purified from this E. coli cell culture medium. The culture fluid was concentrated and desalted by Amicon (membrane YM2) and then the protein was purified by passing through a CM-52 column at pH 7.4 and then eluted with 0.2M NaCl. The PL purified from these simple steps has greater than 95% purity, as judged by electrophoresis on SDS gels followed by staining with Coomassie blue.

### 2.2 CONSTRUCTION OF THE SECRETION VECTOR

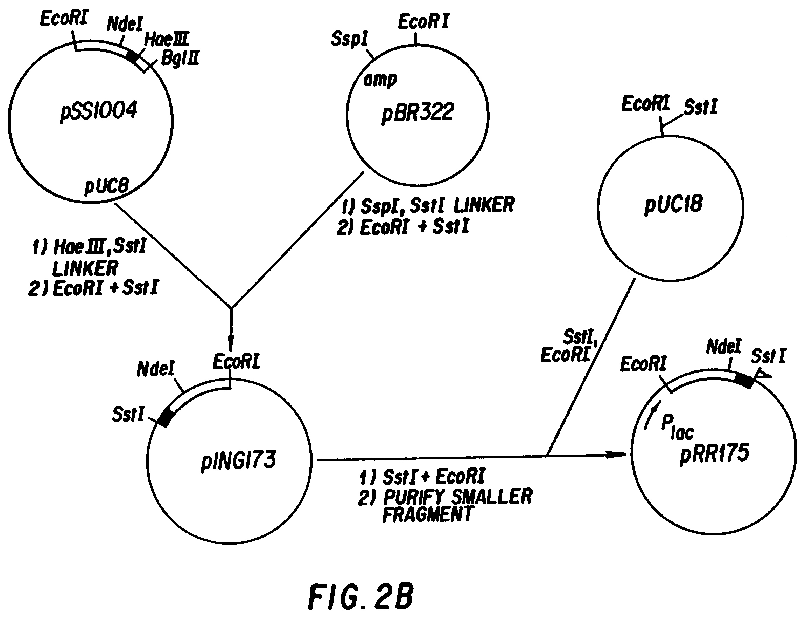

The pSS1004 plasmid was used as the source of the signal sequence for pelB. The sequence was isolated from pSS1004 digestion with restriction enzyme HaeIII, ligation with SstI DNA Linker, and then digestion with EcoRI. The EcoRI-SstI DNA fragment, which contains the 5'-end non-coding region and the leader peptide was then ligated into a pBR322 plasmid digested with SspI and EcoRI. The plasmid so produced containing the signal peptide, is pING173. Figure 2 describes both the DNA sequence for the signal peptide, and the procedure for preparing the pING173 plasmid. This plasmid is used to construct additional derivatives, as described below.

### 2.3. PRODUCTION AND SECRETION OF THAUMATIN

### 2.3.1. BACKGROUND

Thaumatin is a protein sweetener originally isolated from the plant Thaumatococcus danielli. Thaumatin contains 207 amino acids and is 2,000-5,000 fold sweeter than sucrose. It has eight disulfide bonds and the tertiary structure of thaumatin is essential for its biological function.

### 2.3.2. CONSTRUCTION OF A PLASMID CARRYING A PECTATE LYASE B SIGNAL SEQUENCE AND THE SYNTHESIZED PLANT THAUMATIN GENE

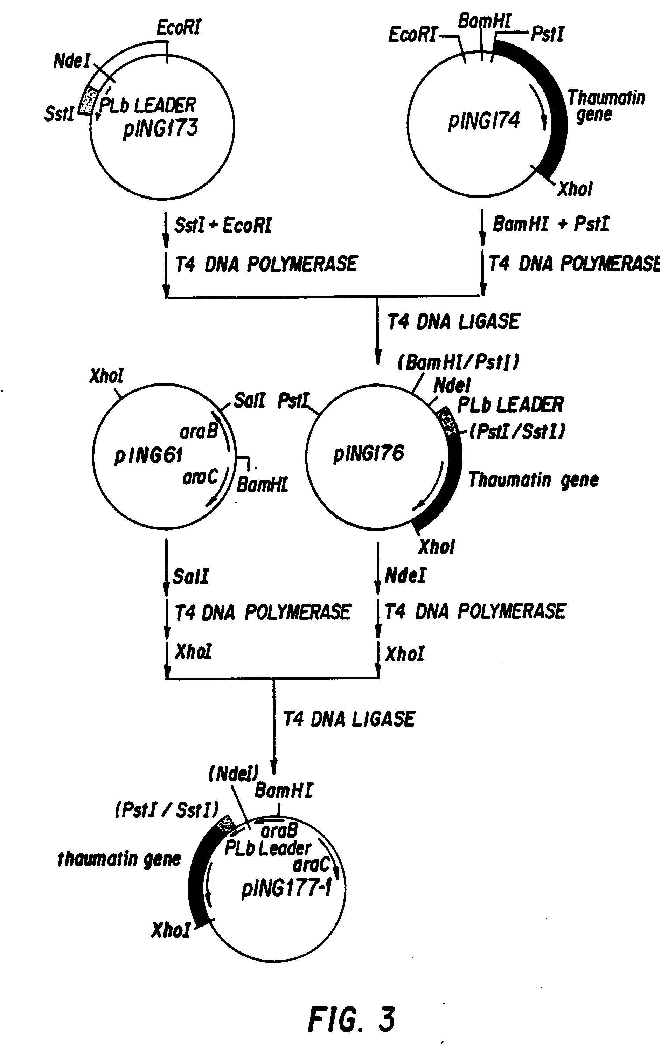

The DNA sequence which codes for PLb signal peptide from plasmid pING173, described above was cloned in front of the thaumatin gene from plasmid pING174 to secrete thaumatin in an E. coli host system. The resulting plasmid, pING177-1, was used to express and secrete thaumatin in E. coli. It has the araBAD promoter and part of the araB gene fused to 50bp of the 5'-non-coding region of the PLb leader peptide. To prepare this plasmid, the SstI and EcoRI fragment of plasmid pING173 was cloned into the plasmid pING174, which contains the thaumatin gene. pING174 was digested with BamHI and PstI, and the leader sequence from pING173 ligated into the restriction sites, to produce pING176. The latter plasmid was digested with NdeI and XhoI, and the resulting fragment, containing the pectate lyase leader sequence adjacent to the thaumatin gene, was cloned into the SalI, XhoI sites on the plasmid pING61. The resulting plasmid contained the gene coding for the PLb leader sequence and the thaumatin gene, under the control of the araBAD promoter and was referred to as pING177-1. The detailed construction scheme is shown in Figure 3.

### 2.3.3. PRODUCTION AND CHARACTERIZATION OF THAUMATIN FROM E. COLI RECOMBINANT STRAIN

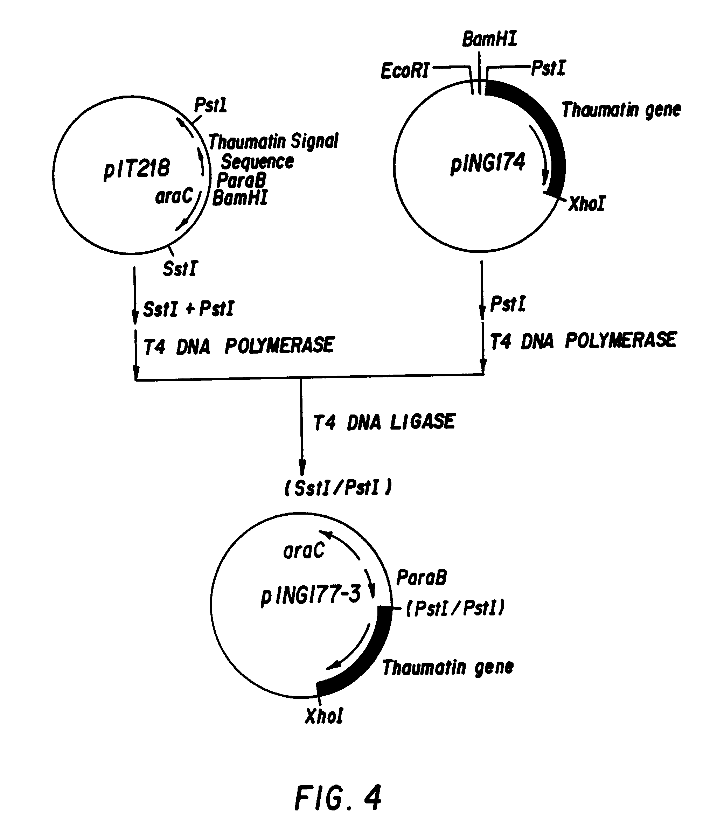

The E. coli strain 706 harboring plasmid pING177-1 was grown in one liter of TYE broth. At O.D.=0.35, the cells were induced with 1% w/v arabinose for approximately 12 hours. The culture was then harvested and the periplasmic space protein was characterized by SDS-PAGE, Western analysis and the RIA assay for properly folded thaumatin. Both SDS-PAGE and Western analysis indicated that thaumatin could be synthesized by E. coli cells. RIA assay also indicated that the secreted thaumatin in the periplasmic space of the E. coli was properly folded (Table 1). The pre-thaumatin signal peptide was also used to secrete thaumatin in E. coli. Plasmid pING177-3, which contains the pre-thaumatin signal peptide sequence and the thaumatin structural gene, was used to produce thaumatin, the detailed construction scheme is shown in Figure 4. The conditions for cell growth and induction are the same as those described previously. The results of the RIA assay indicated that the production of properly-folded thaumatin directed by the pre-thaumatin signal peptide is less efficient than directed by the PLb leader peptide (Table 1).

**TABLE I**

| Secretion of Thaumatin in Escherichia coli (E. Coli K-12; 706) | | | |
|---|---|---|---|
| Plasmid | RIA cross reactive Thaumatin (µg)/gram wet wt. cells | | |
| | Media | Soluble Cell Extract | Total Secreted |
| 177-1 | 5.4 | 35.2 | 40.6 |
| 177-3 | 1.8 | 6.3 | 8.1 |

### 2.4 PRODUCTION AND EXCRETION OF FUNCTIONAL CHIMERIC ANTIBODY

### 2.4.1. BACKGROUND

The Fab molecule of an antibody contains two non-identical protein chains linked by a single disulfide bridge. The two chains are the intact antibody light chain and Fd. The Fd molecule of antibody contains V, J, and CHl portions of the antibody heavy chain. The proper cDNA clones for the L6 chimeric light chain and Fd genes have already been identified. In this example, the sequences coding for PLb leader peptide were used to excrete immunologically active properly assembled Fab of L6 chimeric antibody into the culture growth media.

### 2.4.2. CONSTRUCTION OF E. COLI EXPRESSION AND EXCRETION SYSTEMS FOR L6 CHIMERIC ANTIBODY

The SstI, EcoRI fragment of the plasmid pING173 was cloned into pUC18 to generate pRR175, which contains the pelB leader and adjacent upstream non-coding sequence downstream of the lac promoter. The construction of pRR175 is outlined in Figure 2.

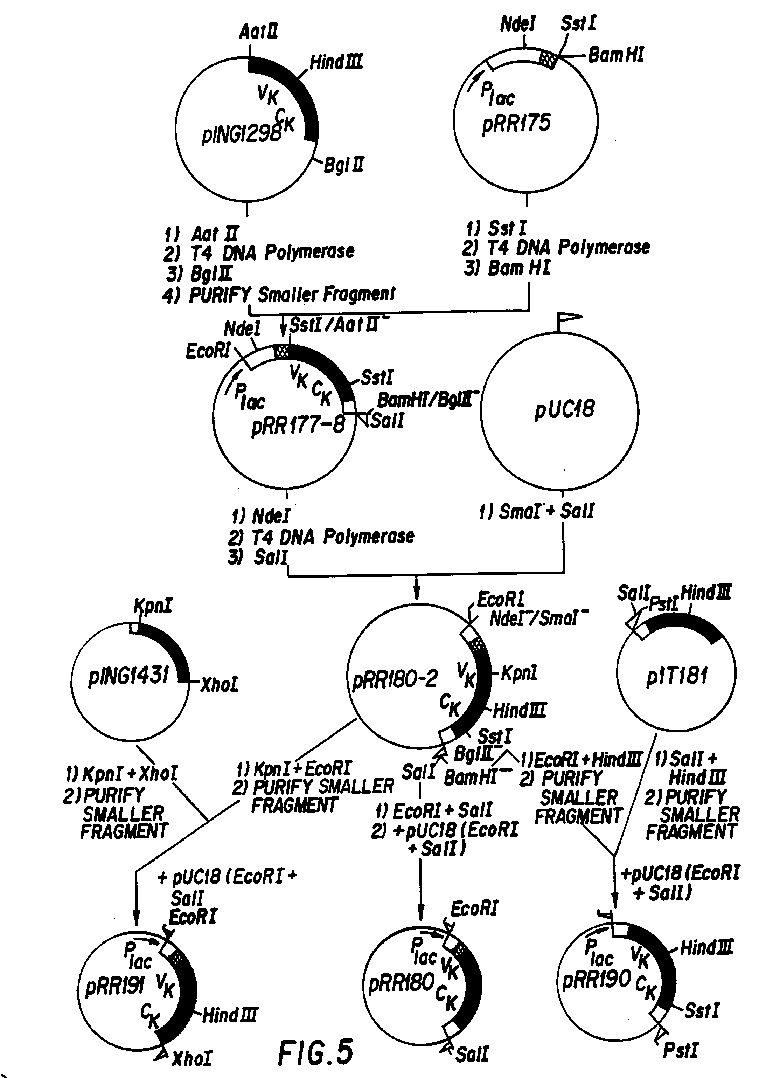

The intact L6 chimeric light chain gene containing an AatII restriction site at the signal sequence processing site and a unique BglII site downstream of the gene was excised from the yeast expression plasmid pING1298 (Figure 5) as a 1200 bp DNA fragment. This fragment was inserted into plasmid pRR175. The resulting plasmid, pRR177-8, contained an in-frame fusion of the pelB leader and the L6 light chain gene downstream of the lac promoter residing in the parent plasmid. A number of derivatives of this plasmid were constructed to delete noncoding sequences from both the 5'- and 3'-ends of the pelB::light chain gene fusion in pRR177-8. Upstream noncoding sequences were deleted making use of an NdeI restriction site at -48 bp from the pelB leader sequence initiation codon (Figure 5) generating pRR180-2. The 3'-noncoding sequences were eliminated by substituting a fragment from the plasmid optimized for L6 light chain expression in yeast, pING1431 (see Figure 5), into pRR180-2 to generate pRR191. These constructions are shown in Figure 5. Another plasmid, pRR190, is similar to pRRl91 but contains 90 bp of noncoding eukaryotic DNA at the 3'-end of the light chain gene.

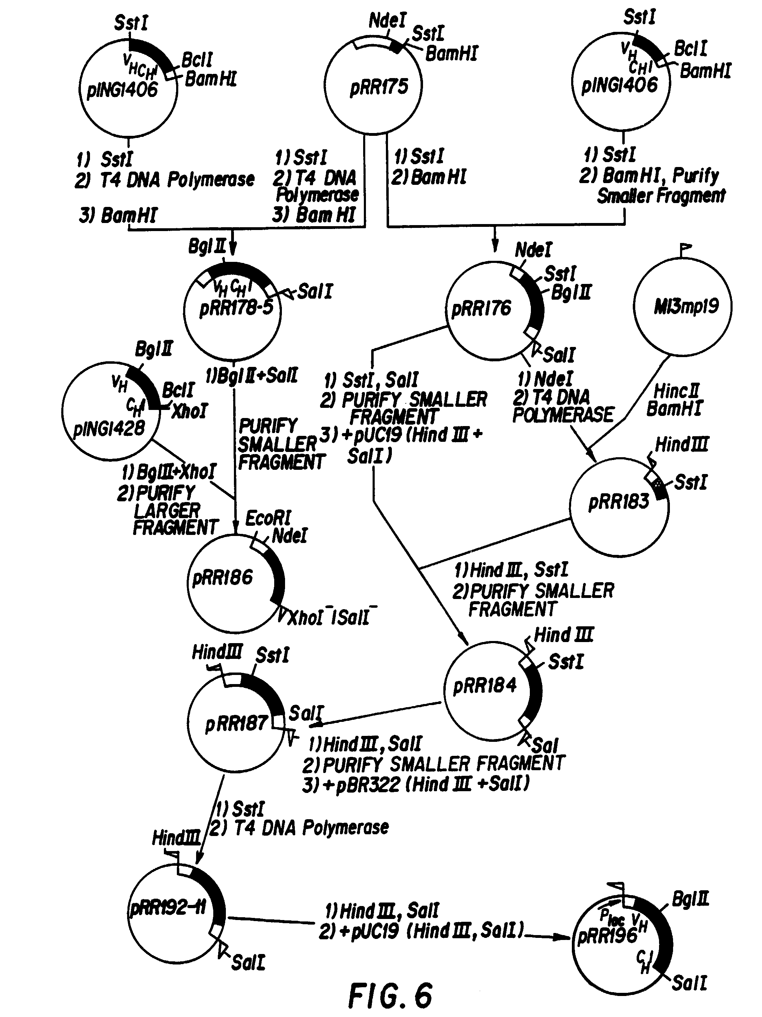

The intact L6 chimeric Fd gene containing an SstI restriction site at the signal sequence processing site, a BclI site introduced by site directed mutagenesis and creating a termination codon at amino acid 226, and a unique BamHI restriction site downstream of the gene was excised from the yeast expression plasmid pING1406 (Figure 6) as a 880 bp DNA fragment. This DNA fragment was inserted into plasmid pRR175 generating an in-frame fusion of the pelB leader sequence and the L6 Fd gene downstream of the lac promoter, pRR178-5. A number of derivatives were constructed to delete noncoding sequences from both the 5'-and 3'-ends of the sequence contained in pRR178-5. The 3'-noncoding sequences were eliminated by substituting a restriction fragment from the plasmid optimized for L6 Fd expression in yeast, pING1428 (Figure 6), which contains an XhoI linker immediately following the termination codon of the Fd gene, generating plasmid pRR186. Removal of E. carotovora DNA sequences upstream of the NdeI site at -48 from the leader sequence generated plasmid pRR196. The construction of these plasmids is shown in Figure 6.

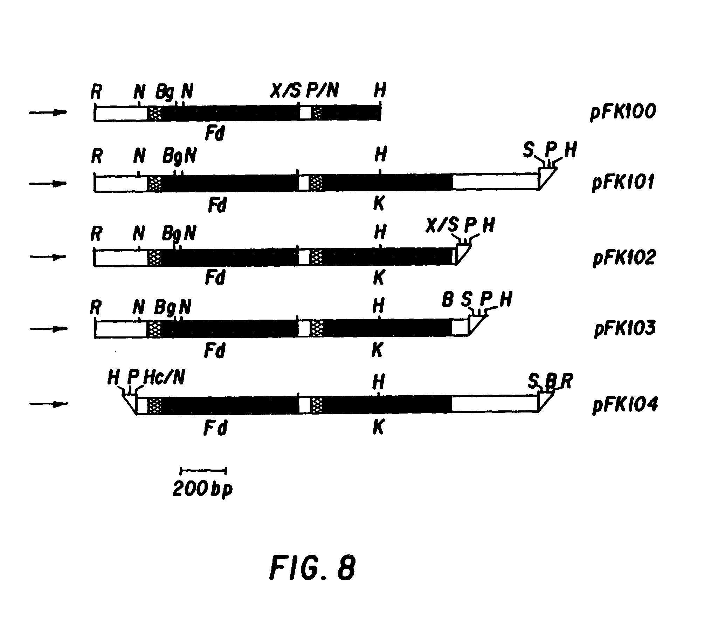

For production of bacterially derived Fab, both light chain and Fd need to be produced simultaneously within the cell. Using the plasmids constructed with each of these genes separately, a series of expression vectors were constructed that contain both genes aligned so that transcription from a single promoter will specify both genes. This was done in a way that minimized the noncoding DNA between the two genes to 60 bp. Each gene has a ribosome binding site needed for translation initiation and the identical DNA sequence from -48 to the pelB leader::antibody gene junction. Several cloning steps were required to align the two genes together. A portion of the light chain gene linked to the pelB leader in pRR180-2 was cloned downstream of the Fd gene in pRR186 to generate pFK100. The remainder of the light chain gene was subcloned into pFKl00 from pRR177-8 to generate pFK101. Similarly, DNA fragments containing 3'- deletions of eukaryotic sequences from pRR190 and pRR191 were cloned into pFK101 generating pFK103 and pFK102 respectively. DNA fragments from pRR192 and pFK101 were ligated to generate pFK104 which contains a deletion of sequences upstream of -48 bp from the Fd gene. Maps of the Fd and light chain gene cassettes in these plasmids are shown in Figure 8. Plasmid pFK102 contains Fd and light chain genes cloned sequentially under the control of the lac promoter in vector pUC18. In E. coli strains such as JM103 F'laciQ (Messing et al., Nucl. Acids. Res. 9: 309, 1981), the amount of light chain that accumulates in the periplasm is not affected by the lac promoter inducing agent isopropyl B-D-thiogalactopyranoside (IPTG). In addition, bacterial growth is slower (compared to cells containing pUC18), and bacterial colonies exhibit an altered morphology being small, dry and rough, suggesting that constitutive foreign gene expression is deleterious to cell growth. Two strategies were used to place this gene cassette under more tightly regulated promoters.

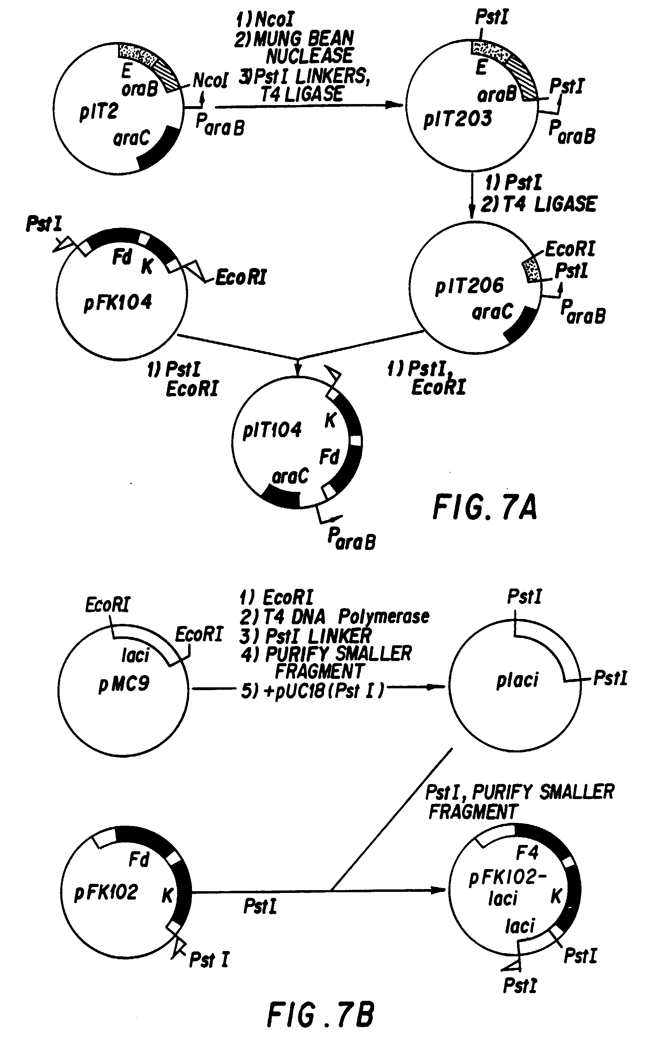

First, a PstI to ECORI fragment from pFK104 was ligated to pIT206 to place the Fd and light chain gene cassette under the direct control of the Salmonella typhimurium araB promoter, a well characterized, strong promoter in E. coli. A restriction map of pIT206 and construction of pITl04 is shown in Figure 7. Use of the araB promoter and its regulatory protein AraC for the expression of bacterial genes is described in U.S. Patent Applications 695,309 filed January 28, 1985, and 797,472, filed November 13, 1985. The resulting plasmid, pIT104, is now regulated for the synthesis of light chain by the addition of arabinose to the culture growth media. At least 10 fold inductions is effected by arabinose addition. Although Fab secreted into the growth medium increases more than 10 fold, cell growth stops after induction with arabinose. This confirms that high level expression of the Fab genes is deleterious to cell growth. Bacterial colonies harboring pIT104 are phenotypically indistinguishable from E. coli harboring pIT206 when grown in the absence of arabinose.

Second, a DNA fragment containing the laci gene, a repressor of the lac promoter, was cloned into the high copy expression vector pFK102. Expression of laci from a high copy number vector is useful to regulate expression of the lac promoter on a high copy number vector (Russel et al., Plasmid, in press (1987); Hsuing et al., Biotechnology 4:991 (1986)). A 1.7 kb EcoRI fragment containing the laci gene on pMC9 S(Calos et al., Proc. Natl. Acad. Sci. USA 80:3015 (1983)) was excised, filled in with T4 polymerase to blunt ends, ligated with PstI linkers and cloned into the unique PstI site of pFK102 to generate pFK102laci. The map of pFK102laci, is shown in Figure 7. The selection procedure used to identify the correct clone assured that the resulting plasmid, pFK102laci, contained a functionally repressed lac promoter. All white or light pink colonies on MacConkey-lactose plates contained plasmids with laci inserts while transformants containing pFK102 alone were red, indicating the functional repression of the lac promoter by the high copy number laci gene. Table III shows that expression of bacterial Fab from cells containing pFK102laci is similar to expression from pFK102. Unlike cells containing pFK102, which formed aberrant colonies and grew slowly in broth culture, cells containing pFK102laci resembled those containing pUC18.

### 2.4.3. EXPRESSION AND PURIFICATION OF BACTERIALLY PRODUCED FAB

A. Growth of E. coli harboring cloned Ab genes Plasmid DNA was transformed into either E. coli JM103 or MC1061 by standard E. coli transformation procedures. Bacterial cultures were grown in TYE (tryptone 1.5%, yeast extract 1.0%, and NaCl 0.5%) supplemented with the appropriate antibiotics (penicillin 250 µg/ml or tetracycline 15 ug/ml). Bacterial cultures were grown in volumes of 5 ml to 1 liter at 37°C to an optical density OD600 = 0.8 (approximately 4 X 10⁸ cell/ml) and aliquots were induced with IPTG (0.2 mM), lactose (1.0%) or arabinose (1.0%). Cultures were grown for an additional time period of 4-21 hours. Portions of each culture were analyzed for light chain production. Protein was released from the periplasmic space of E. coli cells by osmotic shock as described (Yanagida et al. 1986, J. Bacteriol. 166: 937). Alternately, culture supernatants were assayed directly for the presence of antibody chains.

Quantitation of L6 light chain was by ELISA with goat anti-human Kappa light chain antibody (Kappa 1). Fd could be detected by ELISA with mouse monoclonal anti-human Fd antibody (Calbiochem). Table II showns representative data for expression of light chain reactive material in E. coli culture supernatant. This may be a unique property among eukaryotic proteins expressed in E. coli but under certain conditions, bacterial proteins are known to be released from E. coli, (Abrahmsen, et. al. 1986, NAR 14: 7487-7500; Pages, et al. 1986, J. Bacteriol. 169: 1386). Table III compares the amount of light chain secreted in the periplasm with the amount in the culture supernatant. Light chain reactive material is present in plasmid containing cultures harboring cloned light chain alone or light chain plus Fd. The best producers of Fab (pFK102 and pFK102laci) typically excrete 300-1000 ng/ml of ELISA reactive light chain into the culture media.

**TABLE II**

| Quantitation of Light Chain from E. coli Periplasm | | |
|---|---|---|
| | ng/ml of culture | |
| Plasmid | - | + |
| pRR175 | 0 | 0 |
| pRR177-8 | 8.5 | 11 |
| pRR180 | 399 | 412 |

E. coli JM 103 or MC1061 (results similar) was transformed with each plasmid. Fresh transformants were cultured in TYE at 37°C to an OD600 = 0.8. Cultures were divided and the inducer (IPTG) was added to 0.2 mM to one aliquot (- or + IPTG). Cells were grown at 37°C for 4 hours. Periplasmic protein extracts were prepared, and aliquots were tested for light chain by ELISA with goat anti human Kappa antibody. Each value is the average of at least two separate experiments. Removal of non-coding sequences both 5'- and 3'- to the antibody gene effected an increase on light chain accumulation in the periplasm.

**TABLE III**

| Accumulation of Light Chain in the Supernatant and Periplasm after Induction | | | | | |
|---|---|---|---|---|---|
| Plasmid | Inducer | Supernatant | | Periplasm | |
| | | 4 hr | 21 hr | 4 hr | 21 hr |
| pRR190 | - | 0 | nd | 200 | nd* |
| pRR190 | + | 5 | 188 | 241 | nd |
| | | | | | |
| pFK102 | - | 12 | nd | 68 | nd |
| pFK102 | + | 57 | 828 | 55 | 40 |
| | | | | | |
| pFK102laci | - | 25 | 360 | 50 | 100 |
| pFK102laci | + | 72 | 606 | 37 | 40 |
| Plasmid-containing E. coli strains were grown, prepared and assayed. For pRR190, pFK102, and pFK102laci cells were induced with 0.2 mM IPTG. Each value is the average of at least two separate experiments. For analysis of E. coli culture supernatants, bacteria were removed by centrifugation and culture supernatants were passed through a 0.45 µm filter. Values are expressed in ng/ml of culture. *nd - not determined. | | | | | |

### B. SDS-PAGE of Bacterially Produced Light Chain and Fd.

Bacterially produced antibody chains were analyzed by polyacrylamide gel electrophoresis under reducing and non-reducing conditions. Protein extracts of lysed whole bacterial cells, protein released from the periplasmic space by osmotic shock, and protein excreted into the culture supernatant were analyzed. Transfer of gel separated protein under fully reducing conditions to nitrocellulose and immunological staining with goat anti-human light chain antibody by Western analysis revealed that a protein of the same molecular weight as authentic L6 chimeric light chain was present (about 23 Kd). Analysis of protein samples under non-reducing polyacrylamide electrophoresis conditions showed that extracts from cells having a plasmid with the light chain gene alone (pRR191 or pRR190) contained a large proportion of the light chain reactive material associated into a higher molecular weight form. Much of this material ran at about 46 Kd in what is likely to be a light chain dimer. Light chain dimers have been observed from myeloma cells producing only light chain. There are also other immunoreactive protein bands that may represent nonspecific disulfide bond formation between light chain and E. coli proteins. Protein samples (periplasmic extracts or culture supernatants) from E. coli cells harboring both the light chain and Fd genes contain a light chain reactive band at about 48Kd when separated under nonreducing gel conditions which runs at a slightly higher molecular weight than the bacterial light chain dimer. This material is bacterially produced L6 chimeric Fab. In E. coli harboring pFK102laci or pFK102, a 48 Kd band observed on a polyacrylamide gel run under non-reducing conditions is the most prominent immunoreactive species. In addition, the background smear of immunoreactive proteins seen in extracts containing the light chain only is greatly reduced in extracts from cells containing both light chain and Fd.

### C. Purification of Bacterially-Produced Fab

Immunologically and functionally active (see below) bacterial Fab was purified from either culture supernatants or periplasmic protein extracts of E. coli harboring pFK102laci or pIT104. For purification of periplasmic material, the periplasmic fraction from 1 liter of cells induced for 4 hours was released into 50 ml of distilled water. This material was centrifuged for 20 minutes at 5000x g and filtered through a 0.45 um filter. The periplasmic extract was then concentrated over a YM10 membrane (Amicon) to about 5 ml. This material was diluted 8 fold into starting buffer (10 mM K₂HPO₄), pH 7.5) and applied to a 1 ml S-Sepharose column at a flow rate of 1.0 ml/min. The column was washed with 25 ml of starting buffer and eluted with a 0 to 200 mM NaCl gradient in starting buffer (200 ml total volume). The immunoreactive gradient peak was pooled (elution was at about 100 mM) and concentrated on a Centricon 10. Purified Fab was stored in PBS + 2.0% BSA.

For purification of secreted Fab from 1 liter of bacterial culture supernatant, the cells were removed by centrifugation after growth for 21 hours with inducing agents and the supernatant was filtered through a 0.45 um filter. The media was concentrated over a YM10 membrane (Amicon) to about 16 ml, then diluted with 10 mM K₂HPO₄ to 105 ml. This material was applied to a 1.6 ml S-Sepharose column and eluted with a 0 to 200 mM NaCl gradient in 40 ml. Fab recovered from S-Sepharose chromatography was greater than 70% pure as determined by densitometry tracing of a nonreducing, Coomassie stained, 10% acrylamide gel. The molecular weight of Fd and light chain based on the DNA sequence are 24.5 Kd and 23 Kd, which corresponds well to the observed protein sizes. The smaller of the two bands strongly reacted with goat anti-human Kappa light chain antiserum on a Western Blot. Bacterial Fabs purified from either the periplasmic space or bacterial culture supernatants are indistinguishable by all analytical criteria tested here.

### D. Functional Binding Activity of Bacterial L6 Fab to the L6 Antigen.

Bacterially produced Fab purified by S-Sepharose chromatography was tested for binding to L6 antigen containing cells. As shown in Table IV, bacterial Fab binds specifically to the human colon carcinoma cell line H3347. Cells from the T cell line T51 were used as a negative control. Target cells were incubated for 30 minutes at 4°c with bacterially produced L6 chimeric Fab, intact L6 chimeric antibody produced in Sp2/0 cells, or mouse L6 antibody purified from mouse ascites. This was followed by incubation with FITC-labeled goat anti-human light chain antibody for Fab detection, FITC-labeled goat anti-human immunoglobulin for chimeric antibody detection, or with FITC-labeled goat anti-murine immunoglobulin for mouse antibody detection. Antibody binding to the cell surface was determined using a Coulter Model EPIC-C cell sorter. Bacterially produced Fab also exhibits the same characteristic binding inhibition of FITC-labeled mouse L6 antibody to the surface of antigen positive 3347 colon carcinoma cells as the Sp2/0 produced chimeric L6 antibody. Bacterial Fab and Sp2/0 derived chimeric L6 have essentially identical binding inhibition profiles, suggesting the avidity of bacterial Fab and Sp2/0 derived chimeric L6 are the same.

**TABLE IV**

| Binding Assays of Bacterial Fab | | |
|---|---|---|
| Antibody | Binding ratio* | |
| | H3347 cells | T51 cells |
| | L6+ | L6- |
| Standard mouse L6 | 95 | 1 |
| Sp2/0 chimeric L6 | 116 | 1 |
| Bacterial L6 Fab | 54 | 1 |
| Standard L6 Fab | 16 | 1 |

| | | |
|---|---|---|
| *The binding ratio is the number of times brighter a test sample is than a control sample treated with FITC-conjugated second antibody. | | |

Standard L6 Fab was prepared by enzymatic digestion of mouse L6 antibody.

## Claims

1. A recombinant vector suitable for transformation of a bacterial host, the vector containing a DNA sequence coding for a pectate lyase signal peptide adjacent a DNA sequence encoding a protein that is other than:
(a) pectate lyase or
(b) an antibody fragment which is L6 chimeric Fab, L6 chimeric light chain or L6 chimeric Fd; wherein the vector can allow the protein to be expressed and to be transported to the periplasm of a gram-negative host or to be expressed and to be transported extracellularly relative to said host.

2. A vector as claimed in claim 1 which also comprises a DNA promotor sequence which is positioned in relation to the signal and protein sequences so as to permit expression of the protein in the host.

3. A vector as claimed in claim 1 or 2 which is a plasmid.

4. A vector as claimed in any preceding claim wherein the pectate lyase is pectate lyase B of Erwinia carotovora, and/or the promotor is an araBAD or lac promotor.

5. A bacterial host transformed with a vector as claimed in any preceding claim.

6. A host as claimed in claim 5 which is a gram-negative bacterium.

7. A host as claimed in claim 5 or 6 which is E. coli.

8. A method of externalising a protein from the cytoplasm of a host, the method comprising culturing, in an appropriate culture medium, a host as defined in any of claims 5 to 7 which has a promotor DNA sequence positioned in relation to the signal and protein sequences so as to permit expression of the protein in the host.

9. A purified polynucleotide comprising a nucleotide sequence encoding a signal peptide for a pectate lyase and a protein which is other than;
(a) pectate lyase; or
(b) an antibody fragment which is L6 chimeric Fab, L6 chimeric light chain or L6 chimeric Fd;
or mutants or recombinants thereof; wherein said nucleotide sequence when present in a vector in a gram-negative host can allow the protein to be expressed and transported to the periplasm of said host or to be expressed and transported extracellularly relative to said host.

10. A polynucleotide as claimed in claim 9 wherein the pectate lyase is pectate lyase B of Erwinia carotovora.

11. A polynucleotide as claimed in claim 10 wherein the sequence is as depicted in Figure 2A.

## Patentansprüche

1. Rekombinanter Vektor, der zur Transformation eines bakteriellen Wirtes geeignet ist, wobei der Vektor eine für ein Pectatlyase-Signalpeptid codierende DNA-Sequenz angrenzend an eine DNA-Sequenz enthält, die für ein Protein codiert, das ein anderes ist als:
(a) Pectatlyase oder
(b) ein Antikörperfragment, das L6 chimäres Fab, eine L6 chimäre leichte Kette oder L6 chimäres Fd ist,
wobei der Vektor gestatten kann, daß das Protein exprimiert und zum Periplasma eines gramnegativen Wirtes transportiert wird, oder daß es exprimiert und in bezug auf den Wirt extrazellulär transportiert wird.

2. Vektor nach Anspruch 1, der außerdem eine DNA-Promotorsequenz umfaßt, die in bezug zur Signal- und zur Proteinsequenz so angeordnet ist, daß sie die Expression des Proteins in dem Wirt gestattet.

3. Vektor nach Anspruch 1 oder 2, der ein Plasmid ist.

4. Vektor nach einem vorhergehenden Anspruch, bei welchem die Pectatlyase die Pectatlyase B von Erwinia carotovora und /oder der Promotor ein araBAD- oder lac-Promotor ist.

5. Bakterieller Wirt, der mit einem Vektor nach einem vorhergehenden Anspruch transformiert ist.

6. Wirt nach Anspruch 5, der ein gramnegatives Bakterium ist.

7. Wirt nach Anspruch 5 oder 6, der E. coli ist.

8. Verfahren, um ein Protein aus dem Cytoplasma eines Wirtes nach außen zu bringen, wobei das Verfahren die Kultivierung in einem geeigneten Kulturmedium eines Wirtes nach einem der Ansprüche 5 bis 7 umfaßt, der eine Promotor-DNA-Sequenz aufweist, die in bezug zur Signal- und zur Proteinsequenz so angeordnet ist, daß sie die Expression des Proteins in dem Wirt gestattet.

9. Gereinigtes Polynucleotid, das eine Nucleotidsequenz aufweist, die für ein Pectatlyase-Signalpeptid und ein Protein codiert, das ein anderes ist als:
(a) Pectatlyase oder
(b) ein Antikörperfragment, das L6 chimäres Fab, eine L6 chimäre leichte Kette oder L6 chimäres Fd ist,
oder Mutanten oder Rekombinanten davon,
wobei diese Nucleotidsequenz, wenn sie in einem Vektor in einem gramnegativen Wirt vorhanden ist, gestatten kann, daß das Protein exprimiert und zum Periplasma des Wirtes transportiert wird, oder daß es exprimiert und in bezug auf den Wirt extrazellulär transportiert wird.

10. Polynucleotid nach Anspruch 9, wobei die Pectatlyase die Pectatlyase B von Erwinia carotovora ist.

11. Polynucleotid nach Anspruch 10, wobei die Sequenz wie in Figur 2A dargestellt ist.

## Revendications

1. Vecteur recombiné approprié pour la transformation d'un hôte bactérien, le vecteur contenant une séquence d'ADN codant pour un peptide signal de pectate lyase, adjacente à une séquence d'ADN codant pour une protéine autre que :
(a) la pectate lyase, ou
(b) un fragment d'anticorps qui est un Fab chimérique de L6, une chaîne légère chimérique de L6 ou un Fd chimérique de L6;
où le vecteur peut permettre à la protéine d'être exprimée et transportée vers le périplasme d'un hôte Gram négatif ou d'être exprimée et transportée de manière extracellulaire par rapport à l'hôte.

2. Vecteur suivant la revendication 1, qui comprend également une séquence promoteur d'ADN, qui est placée en relation avec les séquences signal et protéique, de manière à permettre l'expression de la protéine dans l'hôte.

3. Vecteur suivant la revendication 1 ou 2, qui est un plasmide.

4. Vecteur suivant l'une quelconque des revendications précédentes, dans lequel la pectate lyase est la pectate lyase B de Erwinia carotovora et/ou le promoteur est un promoteur araBAD ou lac.

5. Hôte bactérien transformé au moyen d'un vecteur suivant l'une quelconque des revendications précédentes.

6. Hôte suivant la revendication 5, qui est une bactérie Gram négative.

7. Hôte suivant la revendication 5 ou 6, qui est E. coli.

8. Procédé d'externalisation d'une protéine à partir du cytoplasme d'un hôte, le procédé comprenant la mise en culture, dans un milieu de culture approprié, d'un hôte tel que défini à l'une quelconque des revendications 5 à 7, qui comprend une séquence promoteur d'ADN placée en relation avec les séquences signal et protéique, de manière à permettre l'expression de la protéine dans l'hôte.

9. Polynucléotide purifié comprenant une séquence nucléotidique codant un peptide signal pour une pectate lyase et une protéine autre que :
(a) la pectate lyase, ou
(b) un fragment d'anticorps qui est un Fab chimérique de L6, une chaîne légère chimérique de L6 ou un Fd chimérique de L6;
ou des mutants ou recombinants de ceux-ci; où la séquence nucléotidique, lorsqu'elle est présente dans un vecteur dans un hôte Gram négatif, peut permettre à la protéine d'être exprimée et transportée vers le périplasme de l'hôte ou d'être exprimée et transportée de manière extracellulaire par rapport à l'hôte.

10. Polynucléotide suivant la revendication 9, dans lequel la pectate lyase est la pectate lyase B de Erwinia carotovora.

11. Polynucléotide suivant la revendication 10, pour lequel la séquence est représentée à la Fig. 2A.
